Europäisches Patentamt

European Patent Office

Office européen des brevets

(11) Publication number: **0 219 716**
**A2**

(12) # EUROPEAN PATENT APPLICATION

(21) Application number: **86113289.2**

(22) Date of filing: **26.09.86**

(51) Int. Cl.⁴: **A 61 K 35/74**

(30) Priority: **26.09.85 JP 210935/85**

(43) Date of publication of application:
**29.04.87 Bulletin 87/18**

(84) Designated Contracting States:
**CH DE FR GB IT LI NL SE**

(71) Applicant: **TOA NENRYO KOGYO KABUSHIKI KAISHA**
**1-1 Hitotsubashi, 1-Chome Chiyoda-Ku**
**Tokyo 100(JP)**

(71) Applicant: **Tsuru, Sumiaki**
**9-7-303, 3-chome, Tagara Nerima-ku**
**Tokyo(JP)**

(72) Inventor: **Tsuru, Sumiaki**
**9-7-303, 3-Chome**
**Tagara, Nerima-ku Tokyo(JP)**

(72) Inventor: **Miki, Keizaburo**
**22-22, 1-Chome**
**Higashiyama, Meguro-ku Tokyo(JP)**

(74) Representative: **Kinzebach, Werner, Dr.**
**Patentanwälte Reitstötter, Kinzebach & Partner**
**Sternwartstrasse 4 Postfach 86 06 49**
**D-8000 München 86(DE)**

(54) Immunosuppressive agent.

(57) An immunosuppressive agent with a strong immunosuppressive effect. The immunosuppressive agent of the present invention comprises as an effective ingredient cholera toxin or an analogue thereof.

EP 0 219 716 A2

Croydon Printing Company Ltd.

SPECIFICATION

TITLE OF THE INVENTION

IMMUNOSUPPRESSIVE AGENT

BACKGROUND OF THE INVENTION

I.   Field of the Invention

This invention relates to an immunosuppressive agent which is used for suppressing rejection reaction in transplanting an organ.

II.  Description of the Prior Art

In transplantation of an organ, the recipient often rejects the transplanted organ due to the immune response of the recipient against the antigens on the transplanted organ.  In order to suppress the rejection reaction, immunosuppressive agent is administered to the recipient. Conventional immunosuppressive agent includes various substances and the most commonly used conventional immunosuppressive agent is cyclosporine A.

Although cyclosporine A is a useful immunosuppressive agent, in some cases, its effect is unsatisfactory.

SUMMARY OF THE INVENTION

Accordingly, the object of the present invention is to provide a novel immunosuppressive agent which has an immunosuppressive effect which is stronger than conventional immunosuppressive agents such as cyclosporine A.

The present invention provides an immunosuppressive agent comprising as an effective ingredient cholera toxin or an analogue thereof.

By the present invention, an immunosuppressive agent of a novel type, which has a strong immunosuppressive effect is provided.

DETAILED DESCRIPTION OF THE PREFERRED EMBODIMENTS

As stated above, the immunosuppressive agent of the present invention comprises as an effective ingredient cholera toxin or an analogue thereof.  Cholera toxin is a known substance which consists of one subunit A with a molecular weight of 27,000 and five subunits B with a molecular weight of 11,600. The amino acid sequences of the subunits have been determined based on the cloned DNAs coding for the subunits of cholera toxin (J.J. Mekalanos et al., Nature 306 pp.551-557 (1983). Cholera toxin is produced by a Gram-negative bacterium Vibrio cholerae. It should be noted, however, cholera toxin produced by other microorganisms or by cultured cells according to a genetic engineering process may also be used. Further, it is well-known by those skilled in the art that, in general, the structure of a protein with a physiological activity is not restricted to only one and single structure, but some other structures which are similar to the structure of the protein with the physiological activity may often express substantially the same physiological activity. Thus, E. coli enterotoxins or modified cholera toxins in which a part of cholera toxin is modified, for example, in a manner that a small number of amino acids are replaced by other amino acids, a small number of amino acids are omitted, or a small number of amino acids are added to the amino acid sequence of the cholera toxin, may also be used if they have the immunosuppressive property. Such enterotoxins and modified cholera toxins with immunosuppressive property are collectively referred to as an "analogue" of cholera toxin in this specification and appended claims.

The most preferred active ingredient of the immunosuppressive agent of the present invention is the cholera toxin in a substantially pure form.

The immunosuppressive agent of the present invention may suitably be used for suppressing the rejection reaction in transplanting various organs such as bone marrow, skin, kidney, heart and liver. It should be noted that the immunosuppressive agent of the present invention may suppress the rejection reaction in transplantation of bone marrow, which is hitherto very difficult to suppress with conventional immunosuppressive agent.

The immunosuppressive agent of the present invention may preferably be administered through a parenteral route, such as, for example, intravenous injection, intraperitoneal injection, intramascular injection and subcutaneous injection.

The immunosuppressive agent of the present invention may preferably be administered before the transplantation of an organ or at the same time as the transplantation, and if necessary, after the transplantation.

The number of times of the administration and the duration of the treatment depends on the organ to be transplanted, the condition of the patient and so on, and they are suitably determined by the doctor. The dose per administration also depends on the organ to be transplanted, the condition of the patient and so on, but usually 0.5 to 50 µg of active ingredient per 1 kg of body weight.

Although the immunosuppressive agent of the present invention may be cholera toxin or an analogue thereof per se, the active ingredient is usually contained in a pharmaceutically acceptable carrier such as physiological saline or buffer solution, which are conventionally used in pharmaceutical compositions. If the carrier is physiological saline or buffer solution, the preferred concentration of the active ingredient may be 0.1 to 2.0 mg/ml. The immunosuppressive agent of the present invention may further contain other pharmaceutically acceptable additives such as, for example, osmoticums, stabilizers and preservatives, which do not adversely affect the immunosuppressive property of the active ingredient.

The $LD_{50}$ of cholera toxin is 5 µg/mouse.

- 4 -

The present invention will now be described in more detail by way of examples. It should be noted that the examples are presented for the illustration purpose only, and they should not be interpreted in any restrictive way.

EXAMPLE 1

Effectiveness for the Transplantation of Bone Marrow (1) Cholera Toxin Used and its Administration Method

Cholera toxin was obtained from <u>Vibrio cholerae</u> in substantially pure form according to the conventional method.

One microgram of cholera toxin was dissolved in 0.2 ml of phosphate buffer, and the cholera toxin solution was administered to female mice (C3H/He) of 7 weeks old through the vein in the tail one day before transplantation.

(2) Bone Marrow Cells Transplanted and Transplantation Method

The bone marrow cells transplanted were obtained from the right and left femora of female C57BL/6 and DBA/2 mice of 7 weeks old.

Immediately after irradiating X-ray of 600 rad. to the bone of the recipients to kill their bone marrow cells, $2 \times 10^7$ bone marrow cells were intravenously injected to each of the recipients.

(3) Criteria for Judging Whether Bone Marrow Cells Were Taken

Whether the bone marrow cells were taken or not was judged according to the survival/death of the recipient, and according to whether the duration in which a skin allograft transplanted to the recipient 20 or 50 days after the transplantation of bone marrow cells was prolonged or not. The transplantation of the skin was conducted by removing 1 cm x 1 cm of whole skin from the body stem of C57BL/6, DBA/2 or BALB/c mice, and by suturing the skin strip to the backside of the right

- 5 -

chest of the recipient.

(4) Results

The results are shown in Tables 1 to 4. As is apparent from the tables, the survival duration of the experimental group was longer than the control group (received nothing) (Table 1), or the duration in which the skin allograft was taken by the recipient (skin-taking duration) was much longer in the experimental groups than in the control groups (Tables 2 to 4), thus showing the immunosuppressive effectivity of the immunosuppressive agent of the present invention is very great.

Table 1

Recipient: C3H/He (H-2$^k$) Mouse

Donor: C57BL/6 (H-2$^b$) Mouse

| Groups | Survival Days |
|---|---|
| Experimental Group (Received Cholera Toxin) | >140,>140,>140,>166,>166,>166 >211,>211,>211 |
| Control Group (Received Nothing) | 18, 19, 21, 22, 28, 30 |

Table 2

Recipient: C3H/He (H-2$^k$) Mouse

Donor of Bone Marrow Cells: C57BL/6 (H-2$^b$) Mouse

Donor of Skin: C57BL/6 (H-2$^b$) Mouse

| Groups | Skin-Taking Days |
|---|---|
| Experimental Group (Received Cholera Toxin) | >114,>114,>132,>187,>187,>187 >210,>210,>210 |

\* Skin was transplanted 50 days after the transplantation of bone marrow cells.

Table 3

Recipient: C3H/He (H-2$^k$) Mouse

Donor of Bone Marrow Cells: C57BL/6 (H-2$^b$) Mouse

Donor of Skin: DBA/2 (H-2$^d$) Mouse

| Groups | Skin-Taking Days |
|---|---|
| Experimental Group (Received Cholera Toxin) | >104,>104,>104,>104,>104,>104 |
| Control Group (Received Nothing) | 7, 7, 8, 7, 6 |

* Skin was transplanted 20 days after the transplantation of bone marrow cells.

Table 4

Recipient: C3H/He (H-2$^k$) Mouse

Donor of Bone Marrow Cells: DBA/2 (H-2$^d$) Mouse

Donor of Skin: BALB/c (H-2$^d$) Mouse

| Groups | Skin-Taking Days |
|---|---|
| Experimental Group (Received Cholera Toxin) | >103,>103,>103,>103,>103,>103 |
| Control Group (Received Nothing) | 7, 7, 7, 6, 6 |

* Skin was transplanted 20 days after the transplantation of bone marrow cells.

EXAMPLE 2

Two Color Analysis by Fluorescence Activated Cell Sortar (FACS)

Bone marrow cells from C57BL/6 mice were transplanted to the test mice in the same manner as in Example 1. Twenty days after the transplantation, the spleen cells of the test mice were double-stained with FITC and phycoerythrin according to the conventional method using anti-H-2K$^k$ antibody and anti-H-2K$^b$$_D$$^b$ antibody.

The results were that, in the experimental group, the percentage of H-2$^{k+}$ or H-2$^{b+}$ cells were 46%, while that of control group (received nothing) was 18%.

EXAMPLE 3

Effectiveness for the Transplantation of Skin Using C3H/He (H-2k) mice as the recipients, and using AKR/N (H-2k) mice and DBA/2 (H-2d) mice as the donors, the duration in which the skin allograft was taken by the recipient was measured. Each mouse in the experimental groups intravenously received 1 μg of cholera toxin in 0.2 ml of phosphate buffer one day before the transplantation, on the day of transplantation, one day after the transplantation, two days after the transplantation, or five days after the transplantation. On the other hand, for the purpose of comparison, 50 mg/kg body weight of cyclosporine A in olive oil was administered every day from the day of transplantation for 14 days. To the control group, skin transplantation was conducted without administering anything. The skin transplantation was conducted in the same manner as in Example 1.

That results were that if the cholera toxin is administered before the transplantation or at the same time as the transplantation, the skin-taking duration was prominently prolonged. That is, when DBA/2 (H-2d) mice were used as the donors, the mean skin-taking days (the duration in which the skin allograft was not dropout) of the experimental group to which cholera toxin was administered was >113.2$\pm$1.0 (standard deviation) (n=11). On the other hand, the mean skin-taking days of the group which received cyclosporine A was 25.2 $\pm$ 2.7 days (n=10), and that of control groups (received nothing) was 17.1$\pm$ 1.6 days (n=8). When AKR/N (H-2k) mice were used as the donors, the mean skin-taking days of the experimental

- 8 -

group which received cholera toxin was >126.2±20.9 days (n=11). On the other hand, the mean skin-taking time of the group which received cyclosporine A was 24.8±0.9 days (n=10), and that of the control group which received nothing was 17.3 ± 1.8 days (n=10).

The results of the experiment in which the cholera toxin was administered to the mice one day before transplantation are summarized in Tables 5 and 6.

Table 5

Recipient:  C3H/Hc (H-2k) Mouse

Donor:  AKR/N (H-2k) Mouse

| Group | Skin-Taking Days |
|---|---|
| Received Cholera Toxin | >117, >117, >117, >110, >117, >110, >117, >117, >110 |
| Received Cyclosporine A | 28, 30, 24, 26, 25, 23, 20, 26, 26, 24 |
| Received Nothing | 18, 17, 16, 14, 17, 19, 18 |

Table 6

Recipient:  C3H/Hc (H-2k) Mouse

Donor:  AKR/N (H-2k) Mouse

| Group | Skin-Taking Days |
|---|---|
| Received Cholera Toxin | >158, >106, >117, >117, >117, >117, >158, >117, >117, >106, >158 |
| Received Cyclosporine A | 26, 24, 26, 24, 25, 26, 24, 25, 24, 24 |
| Received Nothing | 14, 20, 18, 16, 18, 15, 18, 18, 19, 17 |

As shown in the Tables 5 and 6, the immunosuppressive agent of the present invention has a stronger immunosuppressive power than the conventional immunosuppressive agent, cyclosporine A.

EXAMPLE 4

Delayed Footpad Reaction Test

In delayed footpad reaction test, mouse is immunized with an antigen, and the same antigen is injected to the footpad of the mouse 5 to 7 days after the immunization. Due to the immune response of the mouse, the footpad of the mice swells. The swelling reaches to its maximum at 24 hours after the injection. Delayed footpad reaction means this swelling reaction.

Skin was transplanted to the recipient, C3H/He(H-2k) mice from the donor, AKR/N (H-2k) mice or DBA/2 (H-2d) mice. Fourteen days after the transplantation, $1 \times 10^7$ spleen cells from AKR/N (H-2k) mice or DBA/2 (H-2d) mice were injected to the right footpad, and the swelling of the footpad at 24 hours after the injection was determined.

The delayed footpad reaction of the experimental group which received cholera toxin was $1.7 \pm 0.6$ units, while that of control group which received nothing was $4.3 \pm 0.5$ units. It is apparent from these results that the immune response of the mice was significantly suppressed by the immunosuppressive agent of the present invention.

EXAMPLE 5

Mixed Leukocyte Reaction (MLR)

$0.5 \times 10^6$ cells from the spleen of C3H/He (H-2k) mouse were used as the responder. As the stimulator, $0.5 \times 10^6$ spleen cells of AKR/N (H-2k) mouse or DBA/2 (H-2d) mouse, which cells treated with mitomycin C with a final concentration of 20 μg/ml were added to the responder cells. The cell mixture was incubated in a 7%$CO_2$ incubator. Four days after, $^3$H-thymidine was added, and the intake of $^3$H-thymidine by the cells was determined.

The intake of the $^3$H-thymidine by the mouse in which an allograft had long been taken by administering cholera toxin was 14.621 cpm and that of the control mouse was 11.975 cpm, thus there was no significant difference. However, the MLR of the mice which received cholera toxin at one day before measurement was as low as 2.521 cpm.

EXAMPLE 6

## Cell Transfer Test

At the same time as the skin transplantation (Recipient: C3H/He mice, Donor: AKR/N), $1 \times 10^8$ spleen cells from the mice taking skin allograft for a long time[*] or from the mice which received cholera toxin at one day before the collection of the spleen cells, were intravenously injected to the mice. The skin-taking duration of the mice which received the spleen cells from the mice to which cholera toxin was administered was prolonged, while that of the mice which received the spleen cells from the mice taking skin allograft for a long time was not prolonged.

EXAMPLE 7

On the same day as the skin transplantation (Recipient: C3H/He mouse; Donor: AKR/N mouse) and one day after the transplantation, 0.5 ml of blood serum from a mouse taking a skin allograft for a long time and from a mouse to which cholera toxin was administered one day before the transplantation was intraperitoneally administered to the recipient mice. In both cases, the prolongation of the skin-taking time was not observed.

[*] Mouse taking a skin allograft for a long time means a mouse carrying a taken skin allograft for at least 100 days, preventing the dropout of the allograft by administering cholera toxin.

EXAMPLE 8

## Cytotoxic T Lymphocyte Test (CTL)

At the same time as the elicitation in the delayed footpad reaction test, $1 \times 10^7$ spleen cells were intraperitoneally administered to the mice. Three days after the administration, CTL was conducted using as effector cells the glass-nonadherent peritoneal exudate, and using as target cells the concanavalin A blasts labelled with $^{51}$Cr. The amount of $^{51}$Cr released within 4 hours was determined.

In the experimental group which received skin allograft with receiving cholera toxin, the cytotoxic activity was 10% or less, while that of the control group in which the skin transplantation was conducted without administering cholera toxin was about 70%.

- 12 -

Claims

1. An immunosuppressive agent comprising as an effective ingredient cholera toxin or an analogue thereof.

2. The immunosuppressive agent of claim 1, wherein the effective ingredient is cholera toxin in substantially pure form.

3. The immunosuppressive agent of claim 1 or 2, wherein the effective ingredient is contained in a pharmaceutically acceptable carrier.

4. The immunosuppressive agent of claim 3, wherein the pharmaceutically acceptable carrier is physiological saline or a phosphate buffer, and the concentration of the effective ingredient is 0.1 to 2.0 mg/ml.

5. A pharmaceutical for suppressing rejection reaction of a recipient in transplantation of bone marrow, comprising as an effective ingredient cholera toxin or an analogue thereof.

6. The pharmaceutical of claim 5, wherein the effective ingredient is cholera toxin in substantially pure form.

7. The pharmaceutical of claim 5 or 6, wherein the effective ingredient is contained in a pharmaceutically acceptable carrier.

8. The pharmaceutical of claim 7, wherein the pharmaceutically acceptable carrier is physiological saline or a phosphate buffer, and the concentration of the effective ingredient is 0.1 to 2.0 mg/ml.

9. Cholera toxine or an analogue thereof for use in a method for treatment of the human or animal body.

10. The use of cholera toxine or an analogue thereof for preparing an immunosuppressive pharmaceutical.